# EUROPÄISCHE PATENTSCHRIFT

(11) **EP 1 097 144 B1**
(45) Veröffentlichungstag und Bekanntmachung des Hinweises auf die Patenterteilung: **19.03.2008**
(21) Anmeldenummer: 99932871.9
(22) Anmeldetag: 13.07.1999
(51) Int. Cl.: C07D 239/54

(54) **VERFAHREN ZUR HERSTELLUNG 4,6-DISUBSTITUIERTER 2-ISOCYANATOPYRIMIDINE UND IHRE VERWENDUNG ALS ZWISCHENPRODUKTE FÜR WIRKSTOFFSYNTHESEN**
METHOD FOR PRODUCING 4,6-DISUBSTITUTED 2-ISOCYANATOPYRIMIDINES AND THEIR USE AS INTERMEDIATE PRODUCTS FOR ACTIVE INGREDIENT SYNTHESES
PROCEDE DE PRODUCTION DE 2-ISOCYANATOPYRIMIDINES 4,6-BISUBSTITUEES ET LEUR UTILISATION COMME PRODUITS INTERMEDIAIRES POUR LA SYNTHESE DE PRINCIPES ACTIFS

(30) Priorität: 23.07.1998 DE 19833007
(43) Veröffentlichungstag der Anmeldung: 09.05.2001
(73) Patentinhaber: Bayer CropScience AG, 40789 Monheim (DE)
(72) Erfinder: FORD, Mark, James, D-65815 Bad Soden (DE); LACHHEIN, Stephen, D-65719 Hofheim-Wallau (DE)
(86) Internationale Anmeldenummer: PCT/EP1999/004910
(87) Internationale Veröffentlichungsnummer: WO 2000/005220

(56) Entgegenhaltungen:
- EP-A- 0 232 067
- DE-A- 4 206 145
- DE-A- 4 322 726
- US-A- 4 822 402

## Beschreibung

Die Erfindung betrifft das technische Gebiet der chemische Synthese von biologisch aktiven Verbindungen, vorzugsweise der Verfahren zur Herstellung von Pflanzenschutzmitteln und von Zwischenprodukten zu diesen Verfahren.

Es ist bekannt, daß man 4,6-disubstituierte 2-Isocyanatopyrimidine prinzipiell als Zwischenprodukte für die Herstellung von Arzneimitteln, Pflanzenschutzmitteln, Polymeren oder Farbstoffen aus den chemischen Klassen der Carbamate, Harnstoffe und Sulfonylharnstoffe einsetzen kann; vgl. z. B. EP-A-232067, BR-A-8602648 und Handbücher der Chemie. Für die Herstellung der reaktiven IsocyanatGruppe am Pyrimidinrest sind nur wenige Verfahren publiziert.

Nach J. Mass Spec. 30 (1995) S. 338 wurde bei der Hochtemperaturpyrolyse (400-900 °C) bestimmter Sulfonylhamstoffderivate 4,6-Dimethoxy-2-isocyanato-pyrimidin erzeugt und massenspektroskopisch charakterisiert. Das Pyrolyseverfahren hat jedoch nur geringe technische Bedeutung, weil das Produkt damit nicht in nennenswerten präparativen Mengen erhalten werden kann.

In EP-A-232067 ist die Phosgenierung von 2-Amino-4,6-dimethoxy-pyrimidin in Gegenwart einer Aminbase (Triethylamin) beschrieben, wobei das Zwischenprodukt jedoch nicht isoliert oder charakterisiert, sondern direkt mit einem Sulfonamid zu einem Sulfonylharnstoff weiterverarbeitet worden ist. Als Zwischenprodukt wird in der EP-A-232067 gemäß einem allgemeinen Schema 4,6-Dimethoxy-2-isocyanato-pyrimidin und/oder N-(4,6-Dimethoxy-pyrimidin-2-yl)-carbaminsäurechtorid postuliert. Das Verfahren zur Herstellung des Zwischenprodukts als auch das Gesamtverfahren zum herbiziden Sulfonylharnstoff weist jedoch einige Nachteile auf, die gegen eine Durchführung im technischen Maßstab sprechen. Zunächst wird ein großer Überschuß von Aminbase (speziell 4 Äquivalente Triethylamin) und ein großer Überschuß von Phosgen (speziell 8 Äquivalente) eingesetzt. Ein solcher Überschuß läßt sich aus Gründen der Verfahrenssicherheit, Produktqualität und Kostengründen im technischen Maßstab nicht verwenden. Die Produktqualität ist insbesondere beeinträchtigt, weil unter den Bedingungen der Umsetzung und beim Abdestillieren des überschüssigen Phosgens, welches nach EP-A-232067 bei 90 °C durchgeführt wird, die Base Triethylamin und Phosgen miteinander reagieren können (vgl. auch J.-P- Senet, "The Recent Advance in Phosgene Chemistry", Societe Nationale des Poudres et Explosives (Ed.) 1997, S. 105-106). Dies führt einerseits in Abhängigkeit von den im Detail schwer zu kontrollierenden Nebenreaktionen von Reaktionsansatz zu Reaktionsansatz zu schlecht reproduzierbaren Reaktionsverläufen und Ausbeuten sowie zum Teil toxikologisch bedenklichen Nebenprodukten. Beim bekannten Verfahren werden Zersetzungsprodukte und Salze erzeugt, die zur vermehrten Verunreinigung des Produkts beitragen. Außerdem kann das Triethylamin während der Umsetzung durch seinen relativ hohen Dampfdruck mit dem Phosgen in der Gasphase reagieren und einen weißen Niederschlag an verschiedenen Stellen der für die Reaktion verwendeten Apparatur bilden und damit die Reaktionsführung erschweren und die Reinheit des Produkts weiter verschlechtern.

Viele Isocyanate sind sehr reaktiv und werden deshalb nach der Herstellung in der Regel aus dem Reaktionsgemisch oder einer vorgereinigten Lösung nicht isoliert, sondern direkt mit nucleophilen Verbindungen zu Additionsprodukten weiterverarbeitet. Für die Weiterverarbeitung von Isocyanaten des obengenannten Typs sind Lösungsmittel oder Lösungsmittelgemische in unterschiedlicher Weise geeignet. Beispielsweise ist das in EP-A-232067 für die Weiterverarbeitung des Zwischenprodukts eingesetzte Lösungsmittelgemisch nach der Reaktion nur schwer abtrennbar und daher im technischen Maßstab nicht verwendbar. Wegen der genannten Nachteile des bekannten Verfahrens sind dessen Ausbeute für die Herstellung des Zwischenprodukts und dessen Gesamtausbeute für die Herstellung der Weiterverarbeitungsprodukte nicht akzeptabel.

Es bestand daher die Aufgabe, ein modifiziertes Verfahren bereitzustellen, das eine im Vergleich zum obengenannten Verfahren verbesserte bzw. technisch realisierbare Herstellung von 4,6-disubstituierten 2-Isocyanatopyrimidinen bedeutet und vorzugsweise auch eine Weiterverarbeitung zu Carbamaten, Harnstoffen und Sulfonylharnstoffen mit Vorteilen erlaubt, wie verbesserter Gesamtausbeute und/oder Produktreinheit, veringertem Einsatz von Ausgangsmaterialien oder vereinfachtem Verfahrensablauf.

Ein Gegenstand der Erfindung ist ein Verfahren zur Herstellung von Verbindungen der Formel (I), 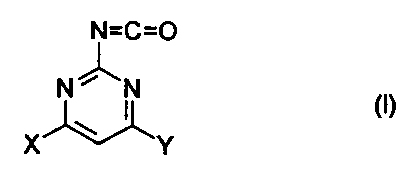 worin jeder der Reste X und Y unabhängig voneinander Wasserstoff, Halogen, (C₁-C₄)Alkyl, (C₁-C₄)Alkoxy oder (C₁-C₄)Alkylthio, wobei jeder der letztgenannten 3 Reste unsubstituiert oder durch einen oder mehrere Reste aus der Gruppe Halogen, (C₁-C₄)Alkoxy und (C₁-C₄)Alkylthio substituiert ist, oder Di[(C₁-C₄)alkyl]-amino, (C₃-C₆)Cycloalkyl, (C₃-C₅)Alkenyl, (C₃-C₅)Alkinyl, (C₃-C₅)Alkenyloxy oder (C₃-C₅)Alkinyloxy bedeutet,
dadurch gekennzeichnet, daß man eine Verbindung der Formel (II) oder deren Salze, 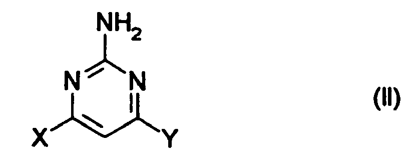 worin X und Y wie in Formel (I) definiert sind,
mit 1 bis 6 Mol Phosgen pro 1 Mol Verbindung der Formel (II), in Gegenwart von 2 bis 3,5 Moläquivalenten einer Base pro Mol Verbindung der Formel (II) und in Gegenwart eines aprotischen organischen Lösungsmittels bei einer Reaktionstemperatur im Bereich von -30 bis +60 °C, vorzugsweise im Bereich von -30 bis +40 °C, insbesondere im Bereich von -10 bis +30 °C zur Verbindung der Formel (I) umsetzt.

Bevorzugt sind die Verfahren zur Herstellung von Verbindungen der Formel (I), worin jeder der Reste X und Y unabhängig voneinander Wasserstoff, Halogen, Methyl, Ethyl, Methoxy, Ethoxy, Methylthio, Trifluormethyl, Trichlormethyl, Difluormethoxy, Dimethylamino, Diethylamino, Allyl, Propargyl, Allyloxy oder Propargyloxy bedeutet;
insbesondere bevorzugt sind dabei solche Verfahren, worin einer der Reste X und Y Halogen, vorzugsweise Chlor, Methyl, Ethyl, Methoxy, Ethoxy, Methylthio, Trifluormethyl, Trichlormethyl, Difluormethoxy, Dimethylamino, Diethylamino, Allyl, Propargyl, Allyloxy oder Propargyloxy bedeutet und der andere der Reste X und Y Methyl, Ethyl, Methoxy, Ethoxy, Methylthio oder Difluormethoxy bedeutet; ganz besonders bevorzugt sind dabei solche Verfahren, worin das X und Y paarweise Methyl/Methyl, Methyl/Methoxy, Chlor/Methyl, Chlor/Methoxy oder Methoxy/Methoxy bedeuten.

Im Zusammenhang mit den in dieser Beschreibung verwendeten chemischen Begriffen gelten die für den Fachmann üblichen Definitionen, sofern nichts anderes spezifisch definiert ist. Die Reste Alkyl, Alkoxy, Haloalkyl, Haloalkoxy, Alkylamino und Alkylthio sowie die entsprechenden ungesättigten und/oder substituierten Reste im Kohlenstoffgerüst jeweils geradkettig oder verzweigt sein. Wenn nicht speziell angegeben, sind bei diesen Resten die niederen Kohlenstoffgerüste, z.B. mit 1 bis 6 C-Atomen bzw. bei ungesättigten Gruppen mit 2 bis 6 C-Atomen, bevorzugt. Alkylreste, auch in den zusammengesetzten Bedeutungen wie Alkoxy, Haloalkyl usw., bedeuten z.B. Methyl, Ethyl, n- oder i-Propyl, n-, i-, t- oder 2-Butyl, Pentyle, Hexyle, wie n-Hexyl, i-Hexyl und 1,3-Dimethylbutyl, Heptyle, wie n-Heptyl, 1-Methylhexyl und 1,4-Dimethylpentyl; Alkenyl- und Alkinylreste haben die Bedeutung der den Alkylresten entsprechenden möglichen ungesättigten Reste; Alkenyl bedeutet z.B. Allyl, 1-Methylprop-2-en-1-yl, 2-Methyl-prop-2-en-1-yl, But-2-en-1-yl, But-3-en-1-yl, 1-Methyl-but-3-en-1-yl und 1-Methyl-but-2-en-1-yl; Alkinyl bedeutet z.B. Propargyl, But-2-in-1-yl, But-3-in-1-yl, 1-Methyl-but-3-in-1-yl.

Cycloalkyl bedeutet ein carbocyclisches, gesättigtes Ringsystem mit vorzugsweise 3-8 C-Atomen, z.B. Cyclopropyl, Cyclobutyl, Cyclopentyl oder Cyclohexyl.

Halogen bedeutet beispielsweise Fluor, Chlor, Brom oder Iod. Haloalkyl, -alkenyl und -alkinyl bedeuten durch Halogen, vorzugsweise durch Fluor, Chlor und/oder Brom, insbesondere durch Fluor und/oder Chlor, teilweise oder vollständig substituiertes Alkyl, Alkenyl bzw. Alkinyl, z.B. Monohaloalkyl (= Monohalogenalkyl), Perhaloalkyl, CF₃, CHF₂, CH₂F, CF₃CF₂, CH₂FCHCl, CCl₃, CHCl₂, CH₂CH₂Cl; Haloalkoxy ist z.B. OCF₃, OCHF₂, OCH₂F, CF₃CF₂O, OCH₂CF, und OCH₂CH₂Cl; entsprechendes gilt für Haloalkenyl und andere durch Halogen substituierte Reste.

Aryl bedeutet ein mono-, bi- oder polycyclisches aromatisches System, beispielsweise Phenyl, Naphthyl, Tetrahydronaphthyl, Indenyl, Indanyl, Pentalenyl, Fluorenyl und ähnliches, vorzugsweise Phenyl.

Ein heterocyclischer Rest oder Ring (Heterocyclyl) kann gesättigt, ungesättigt oder heteroaromatisch sein; er enthält vorzugsweise ein oder mehrere, insbesondere 1, 2 oder 3 Heteroatome im heterocyclischen Ring, vorzugsweise aus der Gruppe N, O, und S; vorzugsweise ist er ein aliphatischer Heterocyclylrest mit 3 bis 7 Ringatomen oder ein heteroaromatischer Rest mit 5 oder 6 Ringatomen. Der heterocyclische Rest kann z.B. ein heteroaromatischer Rest oder Ring (Heteroaryl) sein, wie z.B. ein mono-, bi- oder polycyclisches aromatisches System, in dem mindestens 1 Ring ein oder mehrere Heteroatome enthält, beispielsweise Pyridyl, Pyrimidinyl, Pyridazinyl, Pyrazinyl, Triazinyl, Thienyl, Thiazolyl, Thiadiazolyl, Oxazolyl, Isoxazolyl, Furyl, Pyrrolyl, Pyrazolyl und Imidazolyl, oder ist ein partiell oder vollständig hydrierter Rest wie Oxiranyl, Pyrrolidyl, Piperidyl, Piperazinyl, Dioxolanyl, Oxazolinyl, Isoxazolinyl, Oxazolidinyl, Isoxazolidinyl, Morpholinyl, Tetrahydrofuryl. Als Substituenten für einen substituierten heterocyclischen Rest kommen die weiter unten genannten Substituenten in Frage, zusätzlich auch Oxo. Die Oxogruppe kann auch an den Heteroringatomen, die in verschiedenen Oxidationsstufen existieren können, z.B. bei N und S, auftreten.

Substituierte Reste, wie ein substituierter Alkyl-, Alkenyl-, Alkinyl-, Aryl-, Phenyl-, Benzyl-, Heterocyclyl- und Heteroarylrest, bedeuten beispielsweise einen vom unsubstituierten Grundkörper abgeleiteten substituierten Rest, wobei die Substituenten beispielsweise einen oder mehrere, vorzugsweise 1, 2 oder 3 Reste aus der Gruppe Halogen, Alkoxy, Haloalkoxy, Alkylthio, Hydroxy, Amino, Nitro, Carboxy, Cyano, Azido, Alkoxycarbonyl, Alkylcarbonyl, Formyl, Carbamoyl, Mono- und Dialkylaminocarbonyl, Sulfamoyl, Mono- und Dialkylaminosulfonyl, substituiertes Amino, wie Acylamino, Mono- und Dialkylamino, und Alkylsulfinyl, Haloalkylsulfinyl, Alkylsulfonyl, Haloalkylsulfonyl und, im Falle cyclischer Reste, auch Alkyl und Haloalkyl bedeuten; im Begriff "substituierte Reste" wie substituiertes Alkyl etc. sind als Substituenten zusätzlich zu den genannten gesättigten kohlenwasserstoffhaltigen Resten entsprechende ungesättigte aliphatische und aromatische Reste, wie gegebenenfalls substituiertes Alkenyl, Alkinyl, Alkenyloxy, Alkinyloxy, Phenyl, Phenoxy etc. eingeschlossen. Bei Resten mit C-Atomen sind solche mit 1 bis 4 C-Atomen, insbesondere 1 oder 2 C-Atomen, bevorzugt. Bevorzugt sind in der Regel Substituenten aus der Gruppe Halogen, z.B. Fluor und Chlor, (C₁-C₄)Alkyl, vorzugsweise Methyl oder Ethyl, (C₁-C₄)Haloalkyl, vorzugsweise Trifluormethyl, (C₁-C₄)Alkoxy, vorzugsweise Methoxy oder Ethoxy, (C₁-C₄)Haloalkoxy, Nitro und Cyano. Besonders bevorzugt sind dabei die Substituenten Methyl, Methoxy und Chlor.

Von den Formeln (I) und (II) und auch den Formeln für die Folgeprodukte (siehe weiter unten) sind auch alle Stereoisomeren umfaßt. Solche Verbindungen enthalten ein oder mehrere asymmetrische C-Atome oder auch Doppelbindungen, die in den allgemeinen Formeln nicht gesondert angegeben sind. Die durch ihre spezifische Raumform definierten möglichen Stereoisomeren, wie Enantiomere, Diastereomere, Z- und E-Isomere können nach üblichen Methoden aus Gemischen der Stereoisomeren erhalten oder auch durch stereoselektive Reaktionen in Kombination mit dem Einsatz von stereochemisch reinen Ausgangsstoffen hergestellt werden.

Die erfindungsgemäß einzusetzenden Verbindungen der Formel (II) und deren Salze sind bekannt oder können analog allgemein bekannten Verfahren hergestellt werden (vgl. Literatur zu Vorprodukten für die Herstellung von herbiziden Sulfonylharnstoffen).

Bei der erfindungsgemäßen Umsetzung von Phosgen mit der Aminverbindung der Formel (II) werden gemäß der Stöchiometrie der Reaktion pro 1 Mol umgesetztes Phosgen zwei Mol HCl freigesetzt, die durch die Base gebunden werden sollen. Als Base kommen basische Verbindungen in Frage, die mit dem Isocyanat der Formel (I) unter den Reaktionsbedingungen des erfindungsgemäßen Verfahrens nicht oder im wesentlichen nicht reagieren. Als Basen geeignet sind vor allem organische Aminbasen, wie primäre, sekundäre und tertiäre Amine, insbesondere sterisch gehinderte sekundäre oder vorzugsweise tertiäre Amine.
Geeignete Basen sind aus der Gruppe der Mono-, Di- und Trialkylamine, Mono-, Di- und Tri-aryl-amine, N-Alkyl-N-aryl-amine, N,N-Dialkyl-N-aryl-amine und N-alkyl-N,N-diaryl-amine, wobei jeder der letztgenannten 9 Amine in jedem Alkylteil unabhängig voneinander 1 bis 12 C-Atome, vorzugsweise 1 bis 6 C-Atome, insbesondere 1 bis 4 C-Atome aufweist und wobei jeder der genannten Amine an den Alkylteilen oder Arylteilen unabhängig voneinander unsubstituiert oder weiter mit geeigneten aprotischen Resten substituiert ist.
Geeignete Aminbasen sind auch Amine mit mehreren Aminogruppen, die vorzugsweise sekundäre oder insbesondere tertiäre Aminogruppen enthalten.

Beispiele für einsetzbare Amine sind Trialkylamine bzw. Dialkylaniline wie Trimethylamin, Triethylamin, vorzugsweise N,N-Dimethyl-anilin, N,N-Diisopropyl- N-ethyl-amin oder Tributylamin.

Als Base kann auch teilweise oder ganz die Verbindung der Formel (II) verwendet werden. Dabei sind die Mengenverhältnisse von Phosgen zur Verbindung der Formel (II) erfindungsgemäß im Bereich von 0,33 bis 2 Mol Phosgen pro Mol Verbindung der Formel (II), vorzugsweise 0,33 bis 1 Mol Phosgen, inbesondere 0,33 bis 0,66 Mol Phosgen pro Mol Verbindung der Formel (II).

In der Regel, wird das erfindungsgemäße Verfahren so durchgeführt, daß die Verbindung der Formel (II), vorzugsweise 4,6-Dimethoxy-2-isocyanatopyrimidin, gelöst in einem weitgehend wasserfreien, vorzugsweise wasserfreien aprotischen organischen Lösungsmittel mit 2 bis 3,5 Moläquivalenten, vorzugsweise 2 bis 3 Moläquivalenten, insbesondere 2 bis 2,2 Moläquivalenten Base, jeweils bezogen auf 1 Mol umzusetzende Verbindung der Formel (II) mit Phosgen umgesetzt wird, wobei 1 bis 6, vorzugsweise 1 bis 4, insbesondere 1 bis 3, ganz besonders 1,5 bis 2 Moläquivalente Phosgen pro Mol umzusetzender Verbindung der Formel (II) eingesetzt wird. Das erzeugte Isocyanat der Formel (I) läßt sich in der Regel charakterisieren, z.B. (10% Lösung in Dioxan: IR 2240 cm⁻¹).

Als Lösungsmittel kommen aprotische organische Lösungsmittel in Frage, die unter den Reaktionsbedingungen inert sind, beispielsweise
- aliphatische und aromatische Kohlenwasserstoffe, wie z. B. Mineralöle, Petrolether, Cyclohexan bzw. Toluol, Xylole, Naphthalinderivate, ®Solvesso 200 (hochsiedendes Aromatengemisch);
- halogenierte aliphatische und aromatische Kohlenwasserstoffe, wie Methylenchlorid, Dichlorethan, Chloroform bzw. Chlorbenzol;
- cyclische oder offenkettige Ether, wie Diethylether, Di-n-propylether, Diisopropylether, Methyl-tert-butylether, Tetrahydrofuran (THF), Dioxan, Alkylenglykoldialkylether wie z. B. Propylenglykoldimethylether, Propylenglykoldiethylether, Ethylenglykoldimethylether oder -diethylether, Dimethoxyethan, Diglyme, Triglyme und Tetraglyme;
- Sulfone wie Sulfolan,
- Carbonsäureester, wie die Ester von Mono-, Di- und Tricarbonsäuren mit vorzugsweise 1 bis 4 C-Atomen und aliphatischen (einschließlich cycloaliphatischen) Alkoholen mit 1 bis 10 C-Atomen, beispielsweise Ameisensäureethylester, Essigsäuremethylester, Essigsäureethylester, Essigsäure-n-propylester, Essigsäure-i-propylester, Ester der Essigsäure mit n-, i-, sec.- oder tert.-Butanol,
- Ester der Kohlensäure mit aliphatischen (einschließlich cycloaliphatischen) Alkoholen mit 1 bis 10 C-Atomen, beispielsweise Diethylcarbonat,
- Gemische aus mehreren der vorstehend genannten Lösungsmittel.

Die erfindungsgemäße Umsetzung wird beispielsweise so durchgeführt, daß man Phosgen in eine Lösung oder Suspension der Verbindung der Formel (II) in dem organischen Lösungsmittel, vorzugsweise bei einer Temperatur unter +40 °C, insbesondere unter +30 °C einleitet. Danach kann die Base, vorzugsweise Aminbase pur oder in Form einer Lösung dem organischen Lösungsmittel oder einem Lösungsmittel gleichen Typs bei gleicher Temperatur zugetropft werden. Alternativ kann Phosgen in einem organischen Lösungsmittel vorgelegt und die Verbindung der Formel (II) und die Base nacheinander oder zusammen, pur oder vorzugsweise in Form einer Lösung in dem organischen Lösungsmittel bei vergleichbarer Temperatur zugegeben werden.

Falls eine Verbindung der Formel (II) wie 2-Amino-4,6-dimethoxypyrimidin auch als Base für die Umsetzung benutzt wird, setzt man vorzugsweise 0,33 bis 1 Moläquivalente Phosgen, insbesondere 0,6 bis 0,7 Moläquivalente Phosgen, bezogen auf die Gesamtmenge an Verbindung (II), ein. Die stöchiometrische Ausbeute beträgt bei dieser Verfahrensvariante 0,33 Moläquivalente an Verbindung (I) und 0,66 Moläquivalente an HCl-Salz der Verbindung der Formel (II), jeweils bezogen auf eingesetzte Verbindung (II). In der Regel kann das Salz der Verbindung (II) abfiltriert und die Lösung des Isocyanats weiter verwendet werden und kann aus dem Salz durch Behandlung mit einer starken Base, z. B. wässrige Lösungen von Alkalimetallhydroxiden wie Natronlauge, die Verbindung der Formel (II) als Freibase zurückgewonnen werden.

Ein eventuell eingesetzter Überschuß an Phosgen kann nach der Reaktion beispielsweise durch Durchblasen von Stickstoff, z. B. bei 10 bis 30°C, oder durch Destillation unter Vakuum (Sumpftemperatur vorzugsweise unter 40°C) entfernt werden. Die gebildeten Salze, in der Regel die Aminchlorwasserstoffsalze, können beispielsweise vor oder nach der Entfernung des Phosgens abfiltriert werden. Anschließend kann die Lösung der Verbindung der Formel (I) direkt für Folgeumsetzungen eingesetzt werden. Alternativ kann das Reaktionsgemisch nach Entfernung des Phosgens als Suspension oder als Lösung auch direkt ohne Entsalzung weiter eingesetzt werden.

Die oben beschriebene Herstellung von Isocyanaten der Formel (I) ist überraschenderweise sehr gut reproduzierbar, ergibt in der Regel eine gute bis ausgezeichnete Ausbeute, ermöglicht eine Reduktion des Bedarfs an Phosgen und Aminbase im Vergleich zu dem bekannten Verfahren aus EP-A-232067 mit funktional gleichen bzw. ähnlichen Zwischenprodukten und kann im technischen Maßstab durchgeführt werden.

Die erfindungsgemäß in gelöster Form erhaltenen Verbindungen der Formel (I) können zweckmäßig in an sich bekannter Weise mit Nucleophilen, vorzugsweise protischen Nucleophilen, zu Derivaten unterschiedlichster Art umgesetzt werden. Beispielsweise ermöglicht die Reaktion mit Alkoholen die Herstellung von Carbamaten, die Umsetzung mit primären oder sekundären Aminen ergibt Harnstoffe und die Umsetzung mit Sulfonamiden ergibt Sulfonylharnstoffe.

Gegenstand der Erfindung ist daher auch die Verwendung der erfindungsgemäß erhaltenen Verbindungen der Formel (1) zur Herstellung von Weiterverarbeitungsprodukten und entsprechende Verfahren. Die Weiterverarbeitungsprodukte enthalten vorzugsweise eine Teilstruktur der Verbindung (I), beispielsweise die Teilstruktur der Formel 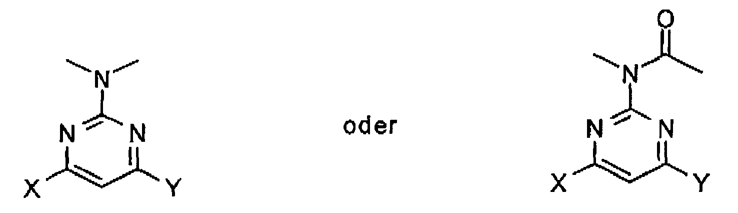 (Hinweis: Die gezeichneten freien Bindungen sollen keine Methylgruppen sein, sondern die Bindungsstellen der Teilstruktur)

Besonders bevorzugt sind dabei die Verfahren zur Herstellung von Verbindungen der Formel (III) 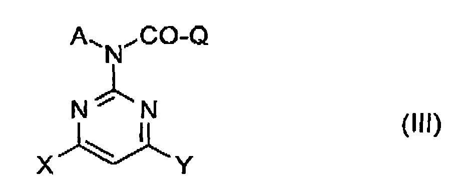 worin X und Y wie in Formel (I) definiert ist und A und Q die weiter unten genannten Bedeutungen haben,
dadurch gekennzeichnet, daß man ein Isocyanat der Formel (I) erfindungsgemäß herstellt und anschließend in an sich bekannter Weise mit Nucleophilen der Formel (IV),

A - Q (IV)

worin
- A: H oder ein Kation und Q ein Rest der Formel R*-Z- bedeutet, worin
- Z: eine divalente Gruppe der Formel -O-, -S-, -NR-, -CO-NR-, -CS-NR-, -SO₂-, -SO₂-NR-, -SO- oder -SO₂-NR-SO₂-, worin R jeweils H oder einen der für R* definierten Reste, vorzugsweise H oder (C₁-C₆)-Alkyl, (C₂-C₆)-Alkenyl, (C₂-C₆)-Alkinyl, (C₃-C₈)-Cycloalkyl, (C₃-C₈)-Cycloalkenyl oder (C₁-C₆)-Alkoxy, besonders bevorzugt H oder Alkyl mit 1 bis 6 C-Atomen, insbesondere Methyl oder Ethyl, bedeutet, und
- R*: einen Rest aus der Gruppe (C₁-C₆)-Alkyl,(C₂-C₆)-Alkenyl, (C₂-C₆)-Alkinyl,(C₃-C₈)-Cycloalkyl, (C₃-C₈)-Cycloalkenyl,(C₁-C₆)-Alkoxy, mono- oder bicyclisches Aryl oder mono- oder bicyclisches Heteroaryl, wobei jeder der letztgenannten 8 Reste unsubstituiert oder substituiert ist, durch einen oder mehrere Reste aus der Gruppe Halogen, (C₁-C₄)-Alkoxy, Halo-(C₁-C₄)-alkoxy, (C₁-C₄)-Alkylthio, Hydroxy, Amino, Nitro, Carboxy, Cyano, Azido, (C₁-C₄)-Alkoxycarbonyl, (C₁-C₄)-Alkylcarbonyl, Formyl, Carbamoyl, Mono- und Di-(C₁-C₄)-Alkylaminocarbonyl, Mono- und Di-(C₁-C₄)-alkylaminosulfonyl, Acylamino, Mono- und Dialkylamino, (C₁-C₄-Alkylsulfinyl, Halo-(C₁-C₄)-alkylsulfinyl, (C₁-C₄)-Alkylsulfonyl und Halo-(C₁-C₄)-alkylsulfonyl und, im Falle cyclischer Reste, auch (C₁-C₄)-Alkyl und Halo-(C₁-C₄)-alkyl,
bedeuten,
an der Isocyanatgruppe zu den Weiterverarbeitungsprodukten (III) umsetzt.

Der Teil Q enthält die nucleophile Gruppe, die an das elektrophile C-Atom der Isocyanatgruppe bindet. In protischen Nucleophilen ist A = Wasserstoff; in nicht protischen Nucleophile ist A von Wasserstoff verschieden, beispielsweise A = ein Kation, z. B. ein Alkalimetallkation wie Natrium- oder Kalium-kation.

Bevorzugt sind als Verbindungen der Formel (IV) die
- Sulfonamide der Formel R¹-SO₂-NH₂
- Sulfonamide der Formel R¹-SO₂-NR-SO₂-NH₂
- Sulfonamide der Formel R¹-NR-SO₂-NH₂
- Sulfonamide der Formel R¹-O-SO₂-NH₂
- Alkohole der Formel R²-OH
- Amine der Formel R³ -NH-R'
(= Formel (IV), worin A = H, Q = R¹-SO₂-NH-, R¹-SO₂-NR-SO₂-NH-, R¹-NR-SO₂-NH-, R²-O- bzw. R³ -NR'-), worin
jeder der Reste R¹, R² und R³ unabhängig voneinander einen Rest aus der Gruppe (C₁-C₆)-Alkyl, (C₂-C₆)-Alkenyl, (C₂-C₆)-Alkinyl,(C₃-C₈)-Cycloalkyl, (C₁-C₆)-Alkoxy, (C₃-C₈)-Cycloalkenyl, mono- oder bicyclisches Aryl oder mono- oder bicyclisches Heteroaryl, wobei jeder der letztgenannten 8 Reste unsubstituiert oder substituiert ist, vorzugsweise unsubstituiert oder durch einen oder mehrere aprotische Reste substituiert ist, und jeder der Reste R und R' unabhängig voneinander einen Rest wie die für R¹, R² oder R³ möglichen Reste oder H, vorzugsweise H oder Alkyl mit 1 bis 6 C-Atomen, bedeuten.

Bevorzugt ist R¹ ein Rest von Sulfonamiden, die für die Herstellung von biologisch aktiven Sulfonylharnstoffen, vorzugsweise Sulfonylharnstoffherbiziden, geeignet sind.
Besonders bevorzugt sind als Verbindungen (IV) die Sulfonamide der Formel R¹-SO₂-NH₂ oder R¹-NR-SO₂-NH₂, worin
- R¹: Phenyl oder mono- oder bicyclisches Heteroaryl, wobei jeder der beiden letztgenannten Reste unsubstituiert oder vorzugsweise durch einen oder mehrere aprotische Reste, vorzugsweise aus der Gruppe Halogen, (C₁-C₄)-Alkyl, Halo-(C₁-C₄)-alkyl, (C₁-C₄)-Alkoxy, Halo-(C₁-C₄)-alkoxy, (C₁-C₄)-Alkylthio, Nitro, Cyano, Azido, (C₁-C₄)-Alkoxycarbonyl, (C₁-C₄)-Alkylcarbonyl, Formyl, Mono- und Dialkyl-(C₁-C₄)-aminocarbonyl, Mono- und Di-(C₁-C₄)-alkylaminosulfonyl, Acylamino, z. B. Acetylamino, Mono- und Dialkylamino, (C₁-C₄)-Alkylsulfinyl, Halo-(C₁-C₄)-alkylsulfinyl, (C₁-C₄)-Alkylsulfonyl und Halo-(C₁-C₄)-alkylsulfonyl substituiert ist, und
- R: H oder (C₁-C₄)Alkyl bedeuten.

Besonders bevorzugt sind auch Sulfonamide der Formel R¹-SO₂-NR-SO₂-NH₂, worin
- R¹: Alkyl bedeutet, das unsubstituiert oder durch einen oder mehrere aprotische Reste aus der Gruppe Halogen, Alkyl, Haloalkyl, Alkoxy, Haloalkoxy, Alkylthio und Phenyl, das unsubstituiert oder substituiert ist, z. B. wie oben für R¹ = Phenyl und Heteroaryl erläutert, substituiert ist,
insbesondere R¹ = (C₁-C₄)Alkyl, und
- R: H oder (C₁-C₄)Alkyl bedeuten.

Bevorzugt sind entsprechende Reste, wie sie in Sulfonamiden zur Herstellung bekannter herbizider Sulfonylharnstoffe bzw. noch bisher unbekannter Verbindungen gleicher Strukturklasse und Wirkungsrichtung eingesetzt werden können (vgl. "The Pesticide Manual", 11th Edition, 1997, British Crop Protection Council und dort zitierte Literatur).

Bevorzugt ist R² ein Rest wie er allgemein für R¹ definiert ist, insbesondere Alkyl; Cycloalkyl oder Phenyl, wobei jeder der letztgenannten 3 Reste unsubstituiert oder durch einen oder mehrere aprotische Reste substituiert ist. Beispiele für Verbindungen der Formel R²OH sind Alkanole, Phenol oder substituierte Phenole. Bevorzugt ist R³ ein Rest wie er für R¹ definiert ist, insbesondere Alkyl, Cycloalkyl oder Phenyl, wobei jeder der letztgenannten 3 Reste unsubstituiert oder durch einen oder mehrere aprotische Reste substituiert ist,
und R' unabhängig voneinander einen Rest wie die für R möglichen Reste oder H, vorzugsweise H oder Alkyl mit 1 bis 6 C-Atomen, bedeutet.

Die Herstellung der Verbindungen der Formel (III) wird in der Regel so durchgeführt, daß man die Verbindungen (I) in Gegenwart eines kleinen Überschusses eines Nucleophils, beispielsweise eines Alkohols oder primären Amins in einem organischen Lösungsmittel, vorzugsweise in dem bei der Herstellung der Verbindung (I) verwendeten organischen Lösungsmittel, gegebenenfalls unter Zusatz einer Base als Katalysator oder zur Salzbildung des Produkts, umsetzt. Als Base kommen dabei nicht nur Aminobasen, sondern auch andere Basen, beispielsweise Metallalkoholate, wie Alkalimetallalkoholate, in Frage.

Die Weiterverarbeitungsreaktion wird vorzugsweise in dem Temperaturbereich durchgeführt, der auch für die Herstellung der Verbindungen (I) geeignet ist, beispielsweise im Bereich von -30 bis +60 °C, vorzugsweise im Bereich von -30 bis +40 °C, insbesondere im Bereich von -10 bis +30 °C.

Zur Herstellung eines Sulfonylharnstoffs kann man beispielsweise das Sulfonamid der Formel (IV) als Feststoff, Flüssigkeit oder in Lösung zur Lösung des isocyanats (I) zugeben und bei dieser Temperatur die Base, z. B. ein Metallalkoholat oder eine Aminbase, pur oder in Lösung zutropfen. Oder aber kann das Isocyanat in Lösung oder Suspension zu einem Gemisch von Sulfonamid (IV) und einer Aminbase oder einem Salz des Sulfonamids, zum Beispiel dem Natrium- oder Kaliumsalz, zugetropft werden.

Die so hergestellten Carbamate, Harnstoffe und Sulfonharnstoffe können durch Methoden, die in Labor und Verfahrenstechnik üblich sind, isoliert und gereinigt werden, z.B. durch Filtration oder Extraktion.

In einer bevorzugten Variante des erfindungsgemäßen Verfahrens wird sowohl in der Stufe der Herstellung der Verbindung (I) als auch bei der Weiterverarbeitung des Isocyanats dasselbe organische Lösungsmittel verwendet, vorzugsweise eines der für das Verfahren zur Herstellung der Verbindung (I) erwähnten bevorzugten organischen Lösungsmittel. Ein Vorteil des erfindungsgemäßen Verfahrens besteht darin, daß eine einfache Verfahrensweise möglich ist und daß die Aufarbeitung überraschenderweise mit besonders guten Ausbeuten verläuft.

Gegebenenfalls kann die Herstellung der Weiterverarbeitungsprodukte auch noch mehrere chemische oder physikalische Verfahrensstufen erfordern.

Die folgenden Beispiele erläutern das erfindungsgemäße Verfahren näher, ohne das erfindungsgemäße Verfahren darauf zu beschänken. In den folgenden Beispielen beziehen sich Mengenangaben auf das Gewicht, sofern nichts anderes speziell definiert ist. Für Maßeinheiten und physikalische Größen werden übliche Abkürzungen verwendet, beispielsweise h = Stunde(n), Smp. = Schmelzpunkt, Fp. = Festpunkt, Schmelzpunkt, I = Liter, g = Gramm, min = Minute(n), i. Vak. = "im Vakuum" = unter reduziertem Druck.

### Beispiele

### 1) N-(4,6-Dimethoxypyrimidin-2-yl)carbaminsäure-isopropylester

In eine Vorlage von Essigsäureethylester (120 ml) wurde Phosgen (20 g, 202 mmol) bei 20-25 °C innerhalb von 45 min eingeleitet. Bei 25°C ließ man eine Lösung von 2-Amino-4,6-dimethoxypyrimidin (15,6 g, 101 mmol) und N,N-Dimethylanilin (26 ml, 203 mmol) in Essigsäureethylester (90 ml) innerhalb von 3 h zutropfen. Nach 15 min wurde die Suspension mit Stickstoff begast, bis sie von Phosgen frei war, wurde Essigsäureethylester (120 ml) zugegeben und Isopropanol (10 ml, 130 mmol) in 10 min unter Kühlung (20-25°C) zugetropft. Die Reaktion wurde nach 1 h abfiltriert, mit Essigsäureethylester gewaschen (3 x 20 ml) und die vereinigten Filtrate mit Salzsäure (1,0 N, 2 x 30 ml) und Wasser (2 x 30 ml) extrahiert. Die Lösung wurde danach konzentriert und das Produkt vom Rückstand kristallisiert; Ausbeute 19,2 g (80% d. Th.), Fp. 51-54°C.

### 2) N-(4,6-Dimethoxypyrimidin-2-yl)carbaminsäure-phenylester

Man verfuhr analog Beispiel 1, wobei jedoch anstelle von Isopropanol eine Lösung von Phenol (10,3 g, 110 mmol) in Essigsäureethylester zugetropft wurde. Man erhielt N-(4,6-Dimethoxypyrimidin-2-yl)carbaminsäure-phenylester in einer Ausbeute von 82 % d. Th..

### 3) N'-(4,6-Dimethoxypyrimidin-2-yl)-N-(2-ethoxyphenoxysulfonyl)-hamstoff

Essigsäureethylester (16 ml) wurde vorgelegt und unter Stickstoff auf -10°C gekühlt. Bei dieser Temperatur wurde Phosgen (4,0g, 0,04 mol) eingeleitet und anschließend eine Lösung von 2-Amino-4,6-dimethoxypyrimidin (2,86 g, 0,0185 mol) und Triethylamin (3,73 g, 0,037 mol) in Essigsäureethylester (20 ml) innerhalb von 2 h zugetropft. Nach 1 h wurde die Reaktion auf 20°C erwärmt, Stickstoff durchgeblasen, bis kein Phosgen mehr in der Lösung war, und dann nacheinander einer Lösung von 2-Ethoxyphenoxysulfonamid (4,0 g, 0,0184 mol) in Essigsäureethylester (10 ml) und Triethylamin (1,86 g, 0,0184 mol) in Essigsäureethylester (10 ml) in 30 min zugetropft. Nach 30 min wurde Wasser (100 ml) zugeben, die Phasen getrennt und die organische Phase mit 2 x 25 ml 1,5 M Natronlauge extrahiert. Die wässrigen Phasen wurden vereinigt, mit Xylol (20 ml) gewaschen, auf pH = 2 mit 6 M Salzsäure eingestellt und das Produkt, abfiltriert und getrocknet; Ausbeute: 5,57g, 70,7% d. Th..

### 4) N,N-Dimethyl-2-{N-[N-(4,6-dimethoxypyrimidin-2-yl)-aminocarbonyl]-aminosulfonyl}-4-nitrobenzamid

Essigsäureethylester (16 ml) und 2-Amino-4,6-dimethoxypyrimidin (5,0 g, 32,2 mmol) wurden vorgelegt und unter Stickstoff auf -10°C gekühlt. Bei dieser Temperatur wurde Phosgen (5,1 g, 51,5 mmol) eingeleitet und eine Lösung von Triethylamin (6,5 g, 64,2 mmol) in Essigsäureethylester (20 ml) innerhalb von 1 Stunde zugetropft. Nach 1 Stunde wurde die Reaktion auf 20°C erwärmt und Stickstoff durchgeblasen, bis sie kein Phosgen mehr enthielt, und dann N,N-Dimethyl-2-aminosulfonyl-4-nitrobenzamid (8,1 g, 29,6 mmol) zugegeben. Das Gemisch wurde auf -10°C gekühlt und eine Lösung von Triethylamin (3,3 g, 32,7 mmol) in Essigsäureethylester (15 ml) in 60 Minuten zugetropft, 30 Minuten nachgerührt und auf Raumtemperatur erwärmt. Kalilauge (1,0 M, 100 ml) wurde dann zugegeben, die Phasen getrennt und die wässrige Phase mit Essigsäureethylester extrahiert. Die wässrige Phase wurde auf pH = 2 mit 6M Salzsäure eingestellt, das Produkt abfiltriert, mit Wasser (2 x 20 ml) gewaschen und getrocknet. Ausbeute: 10,28 g, 72,6% d. Th..

### 5) 2-{N-[N-(4,6-Dimethoxypyrimidin-2-yl)-aminocarbonyl]-aminosulfonyl}-4-cyanobenzoesäuremethylester)

Man verfuhr analog Beispiel 4, wobei jedoch anstelle von N,N-Dimethyl-2-aminosulfonyl-4-nitrobenzamid 2-Aminosulfonyl-4-cyanobenzoesäuremethylester (7,1 g, 29,6 mmol) eingesetzt wurde. Man erhielt 2-{N-(4,6-Dimethoxypyrimidin-2-yl)-aminocarbonyl]-aminosulfonyl}-4-cyanobenzoesäuremethylester in einer Ausbeute von 71 % d.Th.

### 6) N,N-Dimethyl-2-{N-[N-(4,6-dimethoxypyrimidin-2-yl)-aminocarbonyl]-aminosulfonyl}-4-nitrobenzamid

Phosgen (23 g, 232 mmol) wurde in Essigsäureethylester (250 ml) bei 10 °C eingeleitet. Dazu ließ man eine Lösung von 2-Amino-4,6-dimethoxypyrimidin (20 g, 129 mmol) und Tributylamin (47,82 g, 258 mmol) in Essigsäureethylester (270 ml) innerhalb von 3 h zutropfen. 15 min nach der Zugabe wurde der Mantel auf 30 °C erwärmt und Essigsäureethylester und Phosgenüberschuß i. Vak. abdestilliert. Die übriggebliebenen Lösung des Isocyanats wurde auf 10 °C gekühlt und zu einer Suspension von N,N-Dimethyl-2-aminosulfonyl-4-nitrobenzamid (31,7 g, 116 mmol) und Tributylamin (22,96 g, 232 mmol) in Essigsäureethylester (100 ml) bei 20°C innerhalb von 3 h zugetropft. Das Gemisch wurde 1 h nachgerührt und mit Wasser (300 ml) und Kalilauge (10%ig, 240 ml) versetzt. Nach Phasentrennung, wurde die organische Phase mit Wasser (50 ml) extrahiert. Die vereinigten wässrigen Phasen wurden mit Essigsäureethylester (50 ml) extrahiert und mit 6 M Salzsäure auf pH 2 bis 3 eingestellt. Nach Abfiltrieren des Produkts, Waschen mit Wasser (2 x 100 ml) und Trocknen erhielt man 49,09 g (85,8% d. Th.) N,N-Dimethyl-2-{N-[N-(4,6-dimethoxypyrimidin-2-yl)aminocarbonyl]aminosulfonyl}-4-nitrobenzamid.

### Vergleichsbeispiel:

### Herstellung eines Sulfonylharnstoffs analog der Vorschrift aus EP-A-232067

Eine Lösung von 2-Amino-4,6-dimethoxypyrimidin (9,3 g, 60 mmol) und Triethylamin (24,28 g, 240 mmol) in Essigsäureethylester (100 ml) wurde zu einer Lösung von Phosgen (47,11 g, 476 mmol) in Essigsäureethylester (196,3 ml) bei 15°C in 40 Minuten getropft. Es wurde 1 Stunde nachgerührt. Die Mischung wurde auf 90°C erhitzt und das überschüssige Phosgen mit Essigsäureethylester abdestilliert. Der Ansatz wurde auf Raumtemperatur gekühlt und eine Lösung von 2-Aminosulfonyl-4-nitro-N,N-dimethylbenzamid (20,3 g, 74,3 mmol) in Acetonitril (350 ml) in 30 Minuten zugetropft. Anschließend wurde in 1 Stunde Triethylamin (7,2 g, 71,1 mmol) zugetropft und der Ansatz 1,5 Stunden nachgerührt. Das Gemisch wurde dann in Wasser (300 ml) gegossen, die Phasen getrennt und die wässrige Phase mit Salzsäure (18,5%ig) (30 ml) auf pH = 2 eingestellt, das Produkt abfiltriert, mit Wasser (2 x 20 ml) gewaschen und getrocknet. Ausbeute: 21,7 g, 54% d. Th..

## Patentansprüche

1. Verfahren zur Herstellung von Verbindungen der Formel (I), 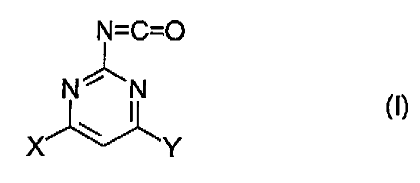 worin jeder der Reste X und Y unabhängig voneinander Wasserstoff, Halogen, (C₁-C₄)Alkyl, (C₁-C₄)Alkoxy oder (C₁-C₄)Alkylthio, wobei jeder der letztgenannten 3 Reste unsubstituiert oder durch einen oder mehrere Reste aus der Gruppe Halogen, (C₁-C₄)Alkoxy und (C₁-C₄)Alkylthio substituiert ist, oder Di[(C₁-C₄)alkyl]-amino, (C₃-C₆)Cycloalkyl, (C₃-C₅)Alkenyl, (C₃-C₅)Alkinyl, (C₃-C₅)Alkonyloxy oder (C₃-C₅)Alkinyloxy bedeutet,
**dadurch gekennzeichnet, daß** man eine Verbindung der Formel (II) oder deren Salze, 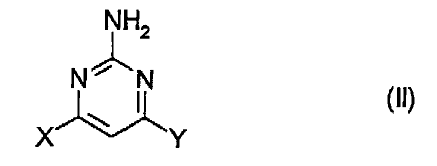 worin X und Y wie in Formel (I) definiert ist,
mit 1 bis 6 Mol Phosgen pro 1 Mol Verbindung der Formel (II), in Gegenwart von 2 bis 3,5 Moläquivalenten einer Base pro Mol Verbindung der Formel (II) und in Gegenwart eines aprotischen organischen Lösungsmittels bei einer Reaktionstemperatur im Bereich von -30 bis +60 °C zur Verbindung der Formel (I) umsetzt.

2. Verfahren nach Anspruch 1, **dadurch gekennzeichnet, daß** X und Y paarweise Methyl/Methyl, Methyl/Methoxy, Chlor/Methyl, Chlor/Methoxy oder Methoxy/Methoxy bedeuten,

3. Verfahren nach Anspruch 1 oder 2, **dadurch gekennzeichnet, daß** man die Umsetzung in Gegenwart von 2 bis 3 Moläquivalenten Base, bezogen auf 1 Mol umzusetzende Verbindung der Formel (II) durchführt.

4. Verfahren nach einem der Ansprüche 1 bis 3, **dadurch gekennzeichnet, daß** eine organische Aminbase als Base eingesetzt wird.

5. Verfahren nach einem der Ansprüche 1 bis 4, **dadurch gekennzeichnet, daß** die Verbindung der Formel (II) auch als Base eingesetzt wird.

6. Verfahren nach einem der Ansprüche 1 bis 5, **dadurch gekennzeichnet, daß** 1 bis 3 Moläquivalente Phosgen pro Mol umzusetzender Verbindung der Formel (II) eingesetzt werden.

7. Verfahren nach Anspruch 6, **dadurch gekennzeichnet, daß** 1,5 bis 2 Moläquivalente Phosgen pro Mol umzusetzender Verbindung der Formel (II) eingesetzt werden.

8. Verfahren nach einem der Ansprüche 1 bis 7, **dadurch gekennzeichnet, daß** ein Lösungsmittel aus der Gruppe aliphatische und aromatische Kohlenwasserstoffe, halogenierte aliphatische und aromatische Kohlenwasserstoffe, cyclische oder offenkettige Ether, Sulfone, Carbonsäureester, Ester der Kohlensäure mit und Gemische aus mehreren der vorstehend genannten Lösungsmittel eingesetzt wird.

9. Verfahren nach Anspruch 8, **dadurch gekennzeichnet, daß** ein Ester von Mono-, Di- und Tricarbonsäuren mit 1 bis 4 C-Atomen und aliphatischen Alkoholen mit 1 bis 10 C-Atomen als Lösungsmittel eingesetzt wird.

10. Verfahren zur Herstellung von Weiterverarbeitungsprodukten von Verbindungen der Formel (I), die in Anspruch 1 definiert ist, **dadurch gekennzeichnet, dass** ein Verfahrensschritt gemäß einem der Ansprüche 1 bis 9 enthalten ist, worin, die zunächst erhaltene Verbindung der Formel (I) in an sich bekannter Weise mit Nucleophilen der Formel (IV),
A - Q (IV)
worin
A Wasserstoff oder ein Kation und
Q einen Rest der Formel R*-Z bedeutet, worin
Z eine divalente Gruppe der Formel -O-, -S-, -NR-, -CO-NR-, -CS-NR-, -SO₂-, -SO₂-NR-, -SO- oder -SO₂-NR-SO₂- bedeutet, worin R jeweils H oder einen der für R* definierten Reste bedeutet, und
R* einen Rest aus der Gruppe (C₁-C₆)Alkyl, (C₂-C₆)Alkenyl, (C₂-C₆)Alkinyl, (C₃-C₈)Cycloalkyl, (C₃-C₈)Cycloalkenyl, (C₁-C₆)Alkoxy, mono- oder bicyclisches Aryl oder mono- oder bicyclisches Heteroaryl bedeutet, wobei jeder der letztgenannten 8 Reste unsubstituiert oder substituiert ist, durch einen oder mehrere Reste aus der Gruppe Halogen, (C₁-C₄)Alkoxy, Halo(C₁-C₄)alkoxy, (C₁-C₄)Alkylthio, Hydroxy, Amino, Nitro, Carboxy, Cyano, Azido, (C₁-C₄)Alkoxycarbonyl, (C₁-C₄)Alkylcarbonyl, Formyl, Carbamoyl, Mono- und Di(C₁-C₄)alkylaminocarbonyl, Sulfamoyl, Mono- und Di(C₁-C₄)alkylarninosulfonyl, Acylamino, Mono- und Dialkylamino, (C₁-C₄)Alkylsulfinyl, Halo(C₁-C₄)alkylsufinyl, (C₁-C₄)Alkylsulfonyl, Halo(C₁-C₄)alkylsulfonyl und, im Falle cyclischer Reste, auch (C₁-C₄)Alkyl und Halo(C₁-C₄)alkyl,
an der Isocyanatgruppe zu Verbindungen der Formel (III), 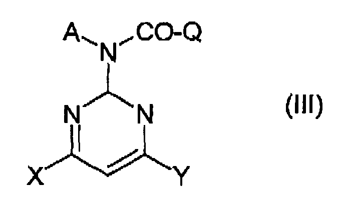 worin X und Y wie in Formel (I) definiert sind und A und Q wie in Formel (IV) definiert sind,
umgesetzt werden.

11. Verfahren zur Herstellung von Weiterverarbeitungsprodukten von Verbindungen der Formel (I), die in Anspruch 1 definiert ist, **dadurch gekennzeichnet, dass** ein Verfahrensschritt gemäß einem der Ansprüche 1 bis 9 enthalten ist, worin, die zunächst erhaltene Verbindung der Formel (I) in an sich bekannter Weise mit Nucleophilen der Formel (IV),
A - Q (IV)
worin
A Wasserstoff und
Q einen Rest der Formel
R¹-SO₂-NH-, R¹-SO₂-NR-SO₂-NH-, R¹-NR-SO₂-NH-, R¹-O-SO₂-NH-, R²-O- oder R³-NR'- bedeutet,
worin
jeder der Reste R¹, R² und R³ unabhängig voneinander einen Rest aus der Gruppe (C₁-C₆)Alkyl, (C₂-C₆)Alkenyl, (C₂-C₆)Alkinyl, (C₃-C₈)Cycloalkyl, (C₁-C₆)Alkoxy, (C₃-C₈)Cycloalkenyl, mono- oder bicyclisches Aryl oder mono- oder bicyclisches Heteroaryl bedeutet, wobei jeder der letztgenannten 8 Reste unsubstituiert oder durch einen oder mehrere aprotische Reste substituiert ist, und jeder der Reste R und R' unabhängig voneinander einen Rest wie die für R¹, R² oder R³ möglichen Reste oder H bedeuten,
an der Isocyanatgruppe zu Verbindungen der Formel (III), 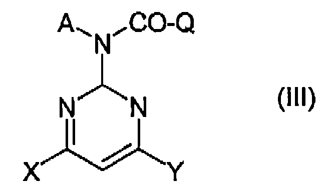 worin X und Y wie in Formel (I) definiert sind und A und Q wie in Formel (IV) definiert sind,
umgesetzt werden.

12. Verfahren nach Anspruch 10 oder 11, **dadurch gekennzeichnet, daß** die Verbindungen der Formel (III) aus der Gruppe der Carbamate, der Harnstoffe oder der Sulfonylharnstoffe sind.

13. Verfahren nach Anspruch 12, **dadurch gekennzeichnet, daß** die Verbindungen der Formel (III) herbizide Sulfonylharnstoffe sind.

## Claims

1. A process for the preparation of compounds of the formula (I) 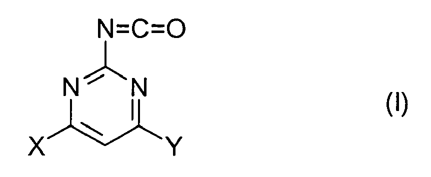 in which each of the radicals X and Y independently of one another is hydrogen, halogen, (C₁-C₄)alkyl, (C₁-C₄)alkoxy or (C₁-C₄)alkylthio, where each of the last-mentioned 3 radicals are unsubstituted or substituted by one or more radicals from the group consisting of halogen, (C₁-C₄)alkoxy and (C₁-C₄)-alkylthio, or di[(C₁-C₄)alkyl]amino, (C₃-C₆)cycloalkyl, (C₃-C₅)-alkenyl, (C₃-C₅)alkynyl, (C₃-C₅)alkenyloxy or (C₃-C₅)-alkynyloxy,
which comprises reacting a compound of the formula (II) or its salts 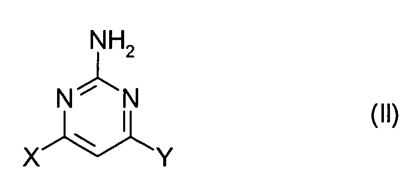 in which X and Y are defined as in formula (I),
with 1 to 6 mol of phosgene per mole of compound of the formula (II), in the presence of 2 to 3.5 molar equivalents of a base per mole of compound of the formula (II) and in the presence of an aprotic organic solvent at a reaction temperature in the range from -30 to +60°C, to give the compound of the formula (I).

2. The process as claimed in claim 1, wherein X and Y in pairs are methyl/methyl, methyl/methoxy, chlorine/methyl, chlorine/methoxy or methoxy/methoxy.

3. The process as claimed in claim 1 or 2, wherein the reaction is carried out in the presence of 2 to 3 molar equivalents of base, relative to 1 mol of compound of the formula (II) to be reacted.

4. The process as claimed in one of claims 1 to 3, wherein an organic amine base is employed as a base.

5. The process as claimed in one of claims 1 to 4, wherein the compound of the formula (II) is also employed as a base.

6. The process as claimed in one of claims 1 to 5, wherein 1 to 3 molar equivalents of phosgene are employed per mole of compound of the formula (II) to be reacted.

7. The process as claimed in claim 6, wherein 1.5 to 2 molar equivalents of phosgene are employed per mole of compound of the formula (II) to be reacted.

8. The process as claimed in one of claims 1 to 7, wherein a solvent from the group consisting of aliphatic and aromatic hydrocarbons, halogenated aliphatic and aromatic hydrocarbons, cyclic or open-chain ethers, sulfones, carboxylic acid esters, esters of carbonic acid with and mixtures of several of the abovementioned solvents is employed.

9. The process as claimed in claim 8, wherein an ester of mono-, di- and tricarboxylic acids having 1 to 4 carbon atoms and aliphatic alcohols having 1 to 10 carbon atoms is employed as a solvent.

10. A process for the preparation of further processing products of compounds of the formula (I) which is defined in claim 1, which contains a process step according to one of claims 1 to 9, wherein the compound of the formula (I) first obtained is reacted in a manner known per se with nucleophiles of the formula (IV)
A - Q (IV)
in which
A is hydrogen or a cation, and
Q is a radical of the formula R*-Z-, in which
Z is a divalent group of the formula -O-, -S-, -NR-, -CO-NR-, -CS-NR-, -SO₂-, -SO₂-NR-, -SO- or -SO₂-NR-SO₂-, in which R is in each case H or one of the radicals defined for R*, and
R* is a radical from the group consisting of (C₁-C₆)alkyl, (C₂-C₆)alkenyl, (C₂-C₆)alkynyl, (C₃-C₈)cycloalkyl, (C₃-C₈)cycloalkenyl, (C₁-C₆)alkoxy, mono- or bicyclic aryl or mono- or bicyclic heteroaryl, where each of the last-mentioned 8 radicals is unsubstituted or substituted, by one or more radicals from the group consisting of halogen, (C₁-C₄)alkoxy, halo(C₁-C₄)alkoxy, (C₁-C₄)alkylthio, hydroxyl, amino, nitro, carboxyl, cyano, azido, (C₁-C₄)alkoxycarbonyl, (C₁-C₄)alkylcarbonyl, formyl, carbamoyl, mono- and di(C₁-C₄)alkylaminocarbonyl, sulfamoyl, mono- and di(C₁-C₄)alkylaminosulfonyl, acylamino, mono- and dialkylamino, (C₁-C₄)alkylsulfinyl, halo(C₁-C₄)alkylsulfinyl, (C₁-C₄)alkylsulfonyl, halo(C₁-C₄)alkylsulfonyl and in the case of cyclic radicals also (C₁-C₄)alkyl and halo(C₁-C₄)alkyl,
at the isocyanate group to give compounds of the formula (III), 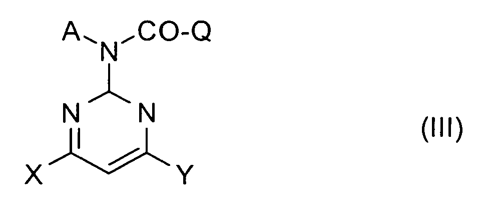 in which X and Y are as defined in formula (I) and A and Q are as defined in formula (IV).

11. A process for the preparation of further processing products of compounds of the formula (I), which is defined in claim 1, which contains a process step according to one of claims 1 to 9, wherein the compound of the formula (I) first obtained is reacted in a manner known per se with nucleophiles of the formula (IV)
A - Q (IV)
in which
A is hydrogen and
Q is a radical of the formula
R¹-SO₂-NH-, R¹-SO₂-NR-SO₂-NH-, R¹-NR-SO₂-NH-, R¹-O-SO₂-NH-, R²-O- or R³-NR'-,
in which
each of the radicals R¹, R² and R³ independently of one another is a radical from the group consisting of (C₁-C₆)alkyl, (C₂-C₆)alkenyl, (C₂-C₆)alkynyl, (C₃-C₈)cydoalkyl, (C₁-C₆)alkoxy, (C₃-C₈)cycloalkenyl, mono- or bicyclic aryl or mono- or bicyclic heteroaryl, where each of the last-mentioned 8 radicals is unsubstituted or substituted by one or more aprotic radicals, and each of the radicals R and R' independently of one another is a radical such as the radicals possible for R¹, R² or R³ or H,
at the isocyanate group to give compounds of the formula (III), 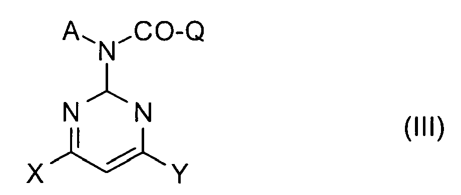 in which X and Y are as defined in formula (I) and A and Q are as defined in formula (IV).

12. The process as claimed in claim 10 or 11, wherein the compounds of the formula (III) are from the group of carbamates, ureas and sulfonylureas.

13. The process as claimed in claim 12, wherein the compounds of the formula (III) are herbicidal sulfonylureas.

## Revendications

1. Procédé de préparation de composés de formule (I), 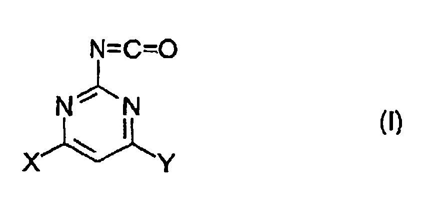 dans laquelle chacun des groupes X et Y représente, indépendamment l'un de l'autre, un atome d'hydrogène, d'halogène, un groupe alkyle(en C₁ à C₄), alcoxyle(en C₁ à C₄) ou alkylthio(en C₁ à C₄), chacun des 3 derniers groupes mentionnés étant non substitué ou substitué par un ou plusieurs groupes choisis dans le groupe constitué par un atome d'halogène, un groupe alcoxyle (en C₁ à C₄) et alkylthio (en C₁ à C₄); ou di[alkyle (en C₁ à C₄)]-amino, cycloalkyle (en C₃ à C₆), alcényle (en C₃ à C₅), alcynyle (en C₃ à C₅), alcényloxy (en C₃ à C₅) ou alcynyloxy (en C₃ à C₅),
**caractérisé en ce que** l'on convertit un composé de formule (II) ou ses sels, 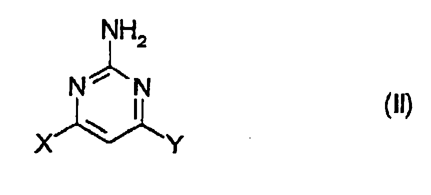 dans laquelle X et Y sont définis comme dans la formule (I),
avec 1 à 6 moles de phosgène pour 1 mole du composé de formule (II), en présence de 2 à 3,5 équivalents molaires d'une base par mole de composé de formule (II) et en présence d'un solvant organique aprotique à une température de réaction dans la gamme de -30°C à +60°C, en composé de formule (I).

2. Procédé selon la revendication 1, **caractérisé en ce que** X et Y représentent des paires méthyle/méthyle, méthyle/méthoxy, chlore/méthyle, chlore/méthoxy ou méthoxy/méthoxy.

3. Procédé selon la revendication 1 ou 2, **caractérisé en ce que** l'on réalise la conversion en présence de 2 à 3 équivalents molaires de base, par rapport à 1 mole du composé de formule (II) à convertir.

4. Procédé selon l'une quelconque des revendications 1 à 3, **caractérisé en ce qu'**une base amine organique est utilisée en tant que base.

5. Procédé selon l'une quelconque des revendications 1 à 4, **caractérisé en ce que** le composé de formule (II) est également utilisé en tant que base.

6. Procédé selon l'une quelconque des revendications 1 à 5, **caractérisé en ce que** 1 à 3 équivalents molaires de phosgène sont utilisés par mole du composé de formule (II) à convertir.

7. Procédé selon la revendication 6, **caractérisé en ce que** 1,5 à 2 équivalents molaires de phosgène sont utilisés par mole du composé de formule (II) à convertir.

8. Procédé selon l'une quelconque des revendications 1 à 7, **caractérisé en ce qu'**un solvant choisi dans le groupe constitué par les hydrocarbures aliphatiques et aromatiques, les hydrocarbures aliphatiques et aromatiques halogénés, les éthers cycliques ou à chaîne droite, les sulfones, les esters d'acides carboxyliques, les esters d'acide carbonique et des mélanges de plusieurs des solvants susmentionnés, est utilisé.

9. Procédé selon la revendication 8, **caractérisé en ce qu'**un ester d'acides mono-, di- et tri-carboxyliques contenant 1 à 4 atomes de carbone et d'alcools aliphatiques contenant 1 à 10 atomes de carbone est utilisé en tant que solvant.

10. Procédé de préparation de produits de transformation de composés de formule (I), qui est définie dans la revendication 1, **caractérisé en ce qu'**il comprend une étape de procédé selon l'une quelconque des revendications 1 à 9, dans laquelle le composé de formule (I) obtenu dans un premier temps est converti de manière connue en soi avec des nucléophiles de formule (IV),
A - Q (IV)
dans laquelle
A représente un atome d'hydrogène ou un cation et
Q représente un groupe de formule R*-Z, où
Z représente un groupe bivalent de formule -O-, -S-, -NR-, -CO-NR-, -CS-NR-, -SO₂-, -SO₂-NR- -SO- ou -SO₂-NR-SO₂-, où R représente à chaque fois un atome d'hydrogène ou un des groupes définis pour R*, et
R* représente un radical choisi dans le groupe constitué par un groupe alkyle(en C₁ à C₆), alcényle (en C₂ à C₆), alcynyle (en C₂ à C₆), cycloalkyle (en C₃ à C₈), cycloalcényle (en C₃ à C₈), alcoxyle (en C₁ à C₆), aryle mono- ou bi-cyclique ou hétéroaryle mono- ou bi-cyclique, chacun des 8 derniers groupes mentionnés étant non substitué ou substitué par un ou plusieurs groupes choisis dans le groupe constitué par un atome d'halogène, un groupe alcoxyle(en C₁ à C₄), halogénoalcoxyle(en C₁ à C₄), alkylthio(en C₁ à C₄), hydroxy, amino, nitro, carboxy, cyano, azido, alcoxy(en C₁ à C₄)carbonyle, alkyl(en C₁ à C₄)carbonyle, formyle, carbamoyle, mono- et di-alkyl(en C₁ à C₄)aminocarbonyle, sulfamoyle, mono- et di-alkyl(en C₁ à C₄)aminosulfonyle, acylamino, mono- et di-alkylamino, alkyl(en C₁ à C₄)sulfinyle, halogénoalkyl(en C₁ à C₄)sulfinyle, alkyl(en C₁ à C₄)sulfonyle, halogénoalkyl(en C₁ à C₄)sulfonyle et, dans le cas de groupes cycliques, également alkyle (en C₁ à C₄) et halogénoalkyle (en C₁ à C₄),
au niveau du groupe isocyanate, en composés de formule (III), 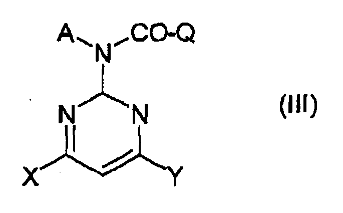 dans laquelle X et Y sont définis comme dans la formule (I) et A et Q sont définis comme dans la formule (IV).

11. Procédé de préparation de produits de transformation de composés de formule (I), qui est définie dans la revendication 1, **caractérisé en ce qu'**il comprend une étape de procédé selon l'une quelconque des revendications 1 à 9, dans laquelle le composé de formule (I) obtenu dans un premier temps est converti de manière connue en soi avec des nucléophiles de formule (IV),
A - Q (IV)
dans laquelle
A représente un atome d'hydrogène et
Q représente un groupe de formule R¹-SO₂-NH-, R¹-SO₂-NR-SO₂-NH-, R¹-NR-SO₂-NH-, R¹-O-SO₂-NH-, R²-O- ou R³-NR',
où
chacun des groupes R¹, R² et R³ représente, indépendamment les uns des autres, un radical choisi dans le groupe constitué par un groupe alkyle (en C₁ à C₆), alcényle (en C₂ à C₆), alcynyle (en C₂ à C₆), cycloalkyle (en C₃ à C₈), alcoxyle (en C₁ à C₆), cycloalcényle(en C₃ à C₈), aryle mono- ou bi-cyclique ou hétéroaryle mono- ou bi-cyclique, chacun des 8 derniers groupes mentionnés étant non substitué ou substitué par un ou plusieurs groupes aprotiques, et chacun des groupes R et R' représente, indépendamment l'un de l'autre, un
groupe comme les groupes possibles pour R¹, R² ou R³ ou un atome d'hydrogène,
au niveau du groupe isocyanate, en composés de formule (III), 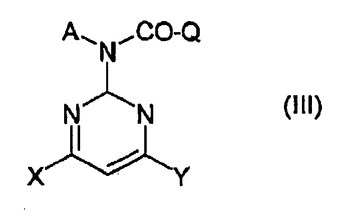 dans laquelle X et Y sont définis comme dans la formule (I) et A et Q sont définis comme dans la formule (IV).

12. Procédé selon la revendication 10 ou 11, **caractérisé en ce que** les composés de formule (III) sont issus du groupe des carbamates, des urées ou des sulfonylurées.

13. Procédé selon la revendication 12, **caractérisé en ce que** les composés de formule (III) sont des sulfonylurées herbicides.
